# FASCICULE DE BREVET EUROPEEN

(11) **EP 3 644 136 B1**
(45) Date de publication et mention de la délivrance du brevet: **08.05.2024**
(21) Numéro de dépôt: 18202782.1
(22) Date de dépôt: 26.10.2018
(51) Int. Cl.: G04G 21/02, A61B 5/00, A61B 5/024

(54) **PROCEDE DE DIFFUSION PAR UNE MONTRE D'UN MESSAGE INFORMATIF RELATIF A UNE EVALUATION DE LA QUALITE DU SOMMEIL D'UN PORTEUR DE LADITE MONTRE**
VERFAHREN ZUR VERBREITUNG ÜBER EINE ARMBANDUHR EINER INFORMATION ÜBER EINE BEWERTUNG DER SCHLAFQUALITÄT EINES TRÄGERS DIESER ARMBANDUHR
METHOD FOR BROADCASTING BY A WATCH OF AN INFORMATION MESSAGE RELATING TO AN EVALUATION OF THE QUALITY OF SLEEP OF A WEARER OF SAID WATCH

(43) Date de publication de la demande: 29.04.2020
(73) Titulaire: Tissot S.A., 2400 Le Locle (CH)
(72) Inventeur: FRANZI, Edoardo, 1400 Cheseaux-Noréaz (CH); DUNBAR, Andrea, 2072 St-Blaise (CH); TÜRETKEN, Engin, 1024 Ecublens (CH); MOSER, Virginie, 2517 Diesse (CH); STADELMANN, Patrick, 2017 Boudry (CH); ZHOU, Lingchuan, 2074 Marin-Epagnier (CH)
(74) Mandataire: ICB SA

(56) Documents cités:
- WO-A2-2007/107900
- WO-A2-2011/109716
- US-A1- 2016 246 259
- US-A1- 2018 279 946

## Description

### Domaine technique

La présente invention concerne un procédé de diffusion par une montre d'un message informatif relatif à une évaluation de la qualité du sommeil d'un porteur de ladite montre et un système mettant en oeuvre un tel procédé.

L'invention concerne également une montre comprenant un tel système ainsi qu'un programme d'ordinateur.

### Art antérieur

Le sommeil est un état durant lequel le corps d'un individu se restaure par exemple en se défendant contre des infections ou encore en fabricant des hormones. La qualité de ce sommeil influe directement sur notre santé mentale, morale et physique. En pareil contexte, on comprend alors qu'il est important de pouvoir évaluer la qualité du sommeil d'un individu afin notamment de pouvoir le cas échéant l'améliorer.

Pour ce faire, on connait dans l'état de la technique des procédés prévoyant de réaliser une évaluation de la qualité du sommeil d'un individu par la mise en oeuvre d'un traitement de données de mesure provenant généralement de capteurs de mouvement ou encore de capteurs physiologiques.

Toutefois un des inconvénients majeurs de tels procédés est lié au fait que l'évaluation de la qualité du sommeil proposée est souvent imprécise voire erronée du fait qu'elle soit réalisée à partir de données de mesure parfois difficiles à obtenir et qui ne sont pas toujours en lien direct avec des conditions liées à la qualité du sommeil. US 2016/246259-A1 divulgue un procédé de diffusion par une montre d'un message informatif relatif à une évaluation de la qualité du sommeil d'un porteur de ladite montre, le procédé comportant les étapes suivantes. US 2018/279946-A1 divulgue la surveillance de la qualité du sommeil d'un utilisateur, et plus spécifiquement, la surveillance des caractéristiques de la qualité du sommeil corrélées avec des facteurs environnementaux, et la fourniture de recommandations automatiques à l'utilisateur.

On comprend qu'il existe un besoin de trouver une solution alternative, notamment qui ne présente pas les inconvénients de l'art antérieur.

### Résumé de l'invention

Un but de la présente invention est par conséquent de proposer un procédé de diffusion par une montre d'un message informatif relatif à une évaluation de la qualité du sommeil qui soit fiable et simple de mise en oeuvre.

Dans ce dessein, l'invention porte sur un procédé de diffusion par une montre d'un message informatif relatif à une évaluation de la qualité du sommeil d'un porteur de ladite montre, selon la revendication 1.

L'invention porte aussi sur un système de diffusion par une montre d'un message informatif relatif à une évaluation de la qualité du sommeil d'un porteur de ladite montre, selon la revendication 7.

L'invention porte en outre sur un programme d'ordinateur selon la revendication 10. Des modes de réalisation préférés sont définis dans les revendications dépendantes 2 à 6, 8, 9.

### Brève description des figures

D'autres particularités et avantages ressortiront clairement de la description qui en est faite ci-après, à titre indicatif et nullement limitatif, en référence aux figures annexées, dans lesquelles :
- la figure 1 est une représentation schématique d'une montre comprenant un système de diffusion d'un message informatif relatif à une évaluation de la qualité du sommeil du porteur de ladite montre, selon un mode de réalisation de l'invention, et
- la figure 2 est un logigramme relatif à un procédé de diffusion de ce message informatif, selon le mode de réalisation de l'invention.

### Description détaillée de l'invention

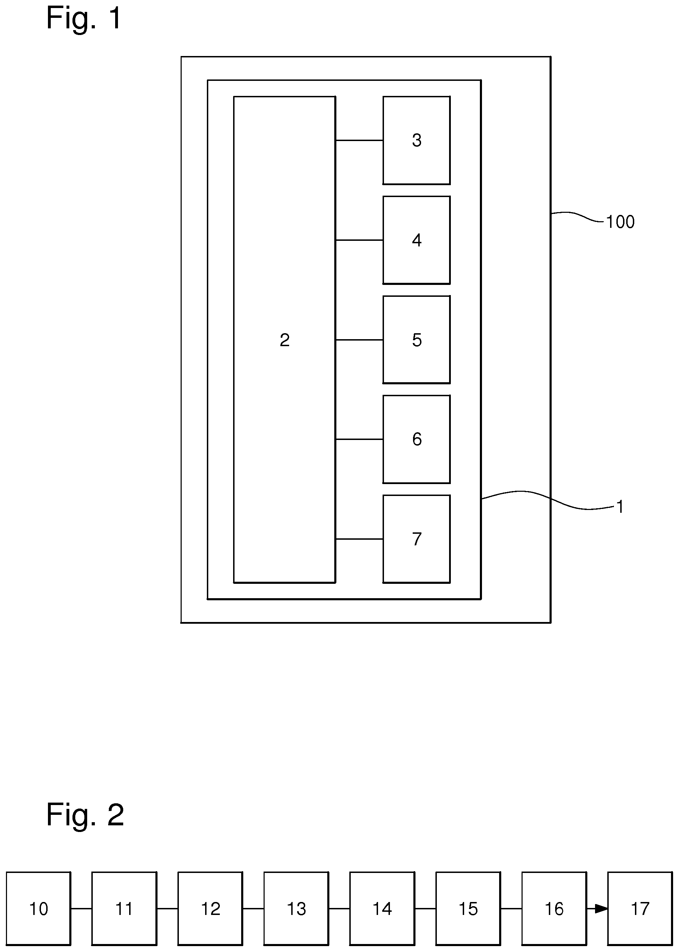

Sur la figure 1 est représentée une montre 100 comprenant le système de diffusion 1 par cette montre 100 d'un message informatif relatif à une évaluation de la qualité du sommeil d'un porteur de ladite montre 100. Un tel système 1 est compris dans la montre 100 qui est de préférence une montre 100 mécanique connecté à affichage hybride. Ce système 1 comprend plus précisément de manière non limitative et/ou non exhaustive :
- une unité de traitement 2 comportant des ressources matérielles et logicielles en particulier au moins un processeur coopérant avec des éléments de mémoire ;
- une interface de diffusion d'une information visuelle 3 telle qu'un cadran d'affichage hybride pourvu d'une première composante d'affichage analogique et d'une deuxième composante d'affichage numérique et/ou alphanumérique ;
- une interface de diffusion d'une information sonore 4 telle qu'un haut-parleur ;
- une interface de communication ;
- au moins un capteur environnemental 5 ;
- au moins un capteur comportemental 6, et
- au moins un capteur physiologique 7.

Dans ce système 1, l'unité de traitement 2 est reliée entre autres aux interfaces de diffusion d'une information visuelle et sonore et aux capteurs environnemental 5, comportemental 6, et physiologique 7.

Le système 1 dans cette montre 100 est apte à évaluer la qualité du sommeil du porteur de préférence uniquement à partir d'au moins un type d'événement environnemental relevé durant une période de sommeil du porteur. Chaque type d'événement relevé est quantifié à partir d'au moins un épisode événementiel qui lui est propre et qui se déroule pendant la période de sommeil du porteur. Chaque épisode événementiel est caractérisé par des données descriptives comprenant notamment une ou plusieurs mesures d'un paramètre environnemental. Ce paramètre environnemental est une grandeur relative à une caractéristique de l'environnement dans lequel est présent la montre 100 et son porteur. Un tel paramètre est de manière non limitative et/ou non exhaustive relatif à : une température, une hygrométrie, un niveau de bruit ambiant, une pression atmosphérique, un éclairement, un mouvement, la qualité de l'air, etc...

A titre d'exemple, lorsque le paramètre correspond à un « niveau de bruit », un épisode événementiel est défini par les données descriptives suivantes :
- une ou plusieurs mesures de ce niveau de bruit sont déterminées par au moins un capteur environnemental idoine 5 et transmises à l'unité de traitement 2 ;
- la durée du déroulement de l'épisode événementiel qui est calculée par l'unité de traitement 2 et est par exemple de 10 minutes, et
- l'heure de début 10 heures du matin et l'heure de fin 10 heures 10 minutes sont déterminées par l'unité de traitement 2.

On comprend qu'à ce stade cet événement environnemental est lié à un « niveau de bruit », et que pour ce type d'événement plusieurs épisodes avec des données descriptives différentes peuvent alors être estimés durant la période de sommeil. Par la suite, selon le procédé décrit plus après, si ce type d'événement environnemental est considéré comme étant perturbateur du sommeil du porteur il devient alors une « nuisance sonore ».

Dans ce contexte, les capteurs environnementaux 5 sont spécifiquement adaptés pour mesurer ces paramètres environnementaux. Ainsi que nous le verrons par la suite les autres capteurs comportementaux 6 et physiologiques 7 participent à la réalisation de mesures qui peuvent être utilisées de manière optionnelle par l'unité de traitement 2 dans le cadre de l'évaluation de la qualité du sommeil du porteur. Les capteurs comportementaux 6 sont aptes à mesurer tous types de caractéristiques comportementales du porteur de la montre 100 comme par exemple les mouvements ou les gestes réalisés par ce dernier pendant la période de sommeil. Pour ce faire, ces capteurs comportementaux 6 peuvent comprendre un ou plusieurs capteurs inertiels de type accéléromètre, gyroscope ou gyromètre multiaxes miniature tels que des capteurs multiaxes fabriqués en technologie MEMS, capables de détecter des vitesses angulaires et des accélérations linéaires selon plusieurs axes associant accéléromètres et/ou gyroscopes. S'agissant des capteurs physiologiques 7, ils sont aptes à mesurer les paramètres relatifs au fonctionnement d'un organisme du porteur tels que, par exemple, le pouls, la saturation du sang en oxygène, l'impédance de la peau, la tension artérielle, le rythme respiratoire, l'arythmie respiratoire, la température cutanée, le taux de sudation, la saturation du sang en oxygène ou le débit sanguin.

Un tel système 1 de la montre 100 est apte à mettre en oeuvre un procédé de diffusion par la montre 100 de ce message informatif représenté sur la figure 2.

Ce procédé comprend une étape d'enregistrement 10 par l'unité de traitement 2 de données descriptives d'au moins un épisode événementiel d'au moins un type d'événement environnemental relevé durant une période de sommeil du porteur. Lors de cette étape 10, dès la détection du début de la période de sommeil du porteur jusqu'à la fin de cette période, les données descriptives d'un ou plusieurs épisodes événementiels d'au moins un type d'événement environnemental sont archivées dans les éléments de mémoire de l'unité de traitement 2 de la montre 100. Ces données descriptives de chaque épisode événementiel comprennent de manière non exhaustive et non limitative :
- une ou plusieurs mesures environnementales déterminées par les capteurs 5 correspondants et transmises à l'unité de traitement 2 ;
- la durée du déroulement de l'épisode événementiel qui est calculée par l'unité de traitement 2, et
- les heures de début et de fin de l'épisode qui sont déterminées par l'unité de traitement 2.

On notera que la création d'un épisode est liée à l'identification par l'unité de traitement 2 d'une variation d'un paramètre environnemental relatif à un type d'événement environnemental correspondant.

Selon l'invention, le début de la période de sommeil peut être déterminé à partir d'une mesure d'éclairement provenant d'un capteur environnemental 5 tel qu'un luxmètre. Ainsi, l'heure de début de la période de sommeil peut être identifiée dès lors qu'une condition d'éclairage faible est alors déterminée par l'unité de traitement 2 par exemple lorsque la mesure d'éclairement transmise par le capteur 5 est inférieure à un seuil d'intensité lumineuse de référence pendant un intervalle de temps déterminé. Alternativement, le début de la période de sommeil peut être détecté à partir de données de mesure physiologique du porteur et/ou de données de mesure comportementale de ce dernier. Par exemple, l'unité de traitement 2 peut déterminer l'heure de début de cette période de sommeil lorsque le capteur comportemental 6 par exemple le capteur inertiel indique que l'utilisateur est immobile pendant un laps de temps déterminé. Dans une variante, l'unité de traitement 2 peut utiliser des informations supplémentaires telles que l'heure du jour et une estimation du rythme circadien du porteur. Par exemple, l'heure de début de veille peut être détectée uniquement pendant une heure déterminée de la journée, lorsque le porteur est supposé s'endormir. De manière similaire, la fin de la période de sommeil peut être déterminé à partir d'une mesure d'éclairement et ce, dès lors qu'une condition d'éclairage forte est alors déterminée par l'unité de traitement 2 par exemple lorsque la mesure d'éclairement transmise par le capteur 5 est supérieure au seuil d'intensité lumineuse de référence pendant un intervalle de temps déterminé. Alternativement, l'unité de traitement 2 peut estimer la fin de la période de sommeil, par exemple, lorsque les données de mesure comportementale relatives au mouvement potentiel du porteur indiquent qu'il s'est levé.

Le procédé comprend ensuite une étape d'identification 11 d'au moins un type d'événement environnemental perturbateur de sommeil du porteur à partir d'un traitement desdites données descriptives. Pour ce faire, cette étape 11 comprend une sous-étape de sélection 12 d'un ou plusieurs épisodes événementiels relatifs à chaque type d'événement environnemental relevé durant la période de sommeil du porteur à partir d'au moins un critère de sélection. Selon un premier critère de sélection, au moins une mesure environnementale des données descriptives de chaque épisode événementiel relatif à chaque type d'événement environnemental, est comparée à un seuil de référence de perturbation du sommeil. Si ladite au moins une mesure est supérieure au seuil de référence de perturbation du sommeil, alors l'épisode peut alors être sélectionné ou présélectionné lorsque les conditions de cette sélection sont liées à la vérification d'un deuxième critère de sélection. Ce deuxième critère de sélection consiste à comparer la durée de chaque épisode relatif à chaque type d'événement environnemental à un seuil de référence de durée de perturbation du sommeil. Dans ce contexte, si la durée de l'épisode est supérieure au seuil de référence de durée de perturbation du sommeil, alors l'épisode est sélectionné.

L'étape d'identification 11 comprend ensuite une sous-étape de détection 13 d'un ou plusieurs types d'événements environnementaux perturbateurs de sommeil du porteur de la montre 100 et ce, à partir des épisodes événementiels qui ont été sélectionnées. Cette sous-étape peut prévoir pour un type d'événement environnemental donné que le nombre d'épisode de cet événement doit être supérieur à un seuil de référence pour être détecté.

Par la suite, l'étape d'indentification 11 comprend une sous-étape de génération 14 d'un indicateur de perturbation du sommeil pour chaque type d'événement environnemental perturbateur de sommeil détecté/identifié. Cette sous-étape 14 comprend une phase 15 de calcul de l'indicateur de perturbation du sommeil pour chaque type d'événement environnemental perturbateur de sommeil identifié à partir de caractéristiques descriptives. Ces caractéristiques descriptives de chaque type d'événement environnemental perturbateur de sommeil identifié sont les suivantes :
- une valeur moyenne du ou des mesures environnementales comprises dans les données descriptives des épisodes événementiels sélectionnés ;
- la durée totale du déroulement de ce type d'événement environnemental perturbateur de sommeil durant la période de sommeil du porteur de la montre 100 qui est égale à la somme des durées des épisodes événementiels sélectionnées ;
- le nombre d'épisodes événementiels sélectionnés ;
- les heures de début et de fin des épisodes événementiels sélectionnés qui permettent d'évaluer leur effet/impact sur la perturbation des cycles du sommeil. En effet, la période de sommeil du porteur comprend une succession de cycles avec des phases de sommeil lent, surtout en début de nuit, qui permet à au corps de récupérer physiquement, et de sommeil paradoxal qui correspond au moment où le porteur rêve et où il évacue sa tension nerveuse.

On notera que le calcul de l'indicateur de perturbation du sommeil est réalisé en fonction d'une ou de plusieurs de ces caractéristiques descriptives.

En outre, une telle étape d'identification 11 peut être mise en oeuvre dès la fin de la période de sommeil ou de manière simultanée à l'étape d'enregistrement 10 c'est-à-dire dès le début de cette période de sommeil.

Le procédé comprend ensuite une étape d'estimation 16 d'un indice d'évaluation de la qualité de ce sommeil en fonction de l'indicateur de perturbation du sommeil de chaque type d'événement environnemental perturbateur de sommeil identifié. Lors de cette étape 16, l'unité de traitement 2 détermine l'indice d'évaluation de la qualité de ce sommeil en calculant une valeur moyenne du ou des indicateurs de perturbation du sommeil obtenus lors de la sous-étape de génération 14. Lors de ce calcul des coefficients peuvent être appliquées à certains indicateurs en fonction de la nature du type de l'événement environnemental perturbateur de sommeil auxquels ils se rapportent. En effet, certains types d'événements peuvent avoir un impact plus important que d'autres sur la perturbation du sommeil.

On notera de manière optionnelle que des données de mesure physiologique et/ou comportementale réalisées durant les épisodes événementiels peuvent également être prises en compte dans le calcul de cet indice.

Ensuite, le procédé comprend une étape de conception 17 du message informatif comprenant l'indice d'évaluation estimé en prévision de sa diffusion à destination du porteur. Un tel message informatif peut être un message sonore ou encore un message visuel comprenant une représentation graphique bidimensionnelle ou tridimensionnelle comportant l'indice. Ce message peut comprendre en plus de l'indice d'évaluation de la qualité du sommeil, une recommandation à l'attention du porteur de la montre 100 relative à une attitude (ou comportement) à adopter pour améliorer cette qualité du sommeil qui est définie en fonction de la valeur de cet indice et donc du niveau de la qualité évaluée du sommeil.

On notera que le procédé peut aussi prévoir une étape durant laquelle, le porteur peut faire part de son évaluation du sommeil en choisissant dans un menu contextuel affiché dans une deuxième composante d'affichage du cadran, un critère relatif à la qualification du sommeil parmi une liste de critères comprenant par exemple les mentions suivantes : une bonne nuit/sommeil, une nuit/sommeil reposante, une nuit/sommeil pénible, etc...

Cette étape prévoit ensuite un archivage de cette évaluation par le porteur de la montre sous la forme de données dont le traitement par l'unité de traitement peut permettre d'améliorer la précision de l'évaluation du sommeil par les présents procédé et système.

L'invention porte aussi sur un programme d'ordinateur comprenant des instructions de code de programme pour l'exécution des étapes 10 à 17 de ce procédé lorsque ledit programme est exécuté par l'unité de traitement 2 de la montre 100.

## Revendications

1. Procédé de diffusion par une montre (100) d'un message informatif relatif à une évaluation de la qualité du sommeil d'un porteur de ladite montre (100), le procédé comportant les étapes suivantes :
- détermination du début de la période de sommeil du porteur :
▪ à partir d'une mesure d'éclairement provenant d'un capteur environnemental (5) de la montre (100), le début de la période de sommeil étant identifiée dès lors qu'une condition d'éclairage faible est alors déterminée par l'unité de traitement (2) de la montre et ce, lorsque la mesure d'éclairement transmise par le capteur (5) est inférieure à un seuil d'intensité lumineuse de référence pendant un intervalle de temps déterminé ; ou
▪ à partir de données de mesure physiologique du porteur et/ou de données de mesure comportementale de ce dernier; ou
▪ à partir de l'utilisation par l'unité de traitement (2) d'informations supplémentaires telles que l'heure du jour et une estimation du rythme circadien du porteur ;
- en réponse à la détermination du début de la période de sommeil du porteur enregistrement (10), par l'unité de traitement (2), de données descriptives d'au moins un épisode événementiel d'au moins un type d'événement environnemental relevé durant ladite période de sommeil du porteur ;
- identification (11) d'au moins un type d'événement environnemental perturbateur de sommeil du porteur à partir d'un traitement desdites données descriptives ;
- estimation (16) d'un indice d'évaluation de la qualité du sommeil du porteur en fonction d'un indicateur de perturbation du sommeil de chaque type d'événement environnemental perturbateur de sommeil identifié, dans laquelle, lors de cette étape (16) l'unité de traitement (2) détermine l'indice d'évaluation de la qualité de ce sommeil en calculant une valeur moyenne du ou des indicateurs de perturbation du sommeil obtenus d'une sous-étape de génération (14) d'un indicateur de perturbation du sommeil pour chaque type d'événement environnemental perturbateur de sommeil identifié, durant ce calcul des coefficients peuvent être appliquées à certains indicateurs en fonction de la nature du type de l'événement environnemental perturbateur de sommeil auxquels ils se rapportent ; et
- conception (17) du message informatif comprenant l'indice d'évaluation estimé en prévision de sa diffusion à destination du porteur.

2. Procédé selon la revendication précédente, **caractérisé en ce que** l'étape d'identification (11) comprend une sous-étape de sélection (12) d'un ou de plusieurs épisodes événementiels relatifs à chaque type d'événement environnemental relevé durant la période de sommeil du porteur à partir d'au moins un critère de sélection.

3. Procédé selon la revendication précédente, **caractérisé en ce qu'**un premier critère de sélection prévoit qu'au moins une mesure environnementale des données descriptives de chaque épisode événementiel relatif à chaque type d'événement environnemental est comparée à un seuil de référence de perturbation du sommeil.

4. Procédé selon l'une quelconque des revendications 2 et 3, **caractérisé en ce qu'**un deuxième critère de sélection consiste à comparer une durée de chaque épisode événementiel relatif à chaque type d'événement environnemental à un seuil de référence de durée de perturbation du sommeil.

5. Procédé selon l'une quelconque des revendications précédentes, **caractérisé en ce que** l'étape d'identification (11) comprend une sous-étape de génération (14) d'un indicateur de perturbation du sommeil pour chaque type d'événement environnemental perturbateur de sommeil identifié.

6. Procédé selon la revendication précédente, **caractérisé en ce que** la sous-étape de génération (14) comprend une phase (15) de calcul de l'indicateur de perturbation du sommeil pour chaque type d'événement environnemental perturbateur de sommeil identifié à partir des caractéristiques descriptives suivantes :
- une valeur moyenne du ou des mesures environnementales comprises dans les données descriptives des épisodes événementiels sélectionnés relatifs à ce type d'événement environnemental ;
- une durée totale du déroulement de ce type d'événement environnemental ;
- le nombre d'épisodes événementiels sélectionnés de ce type d'événement environnemental ;
- des heures de début et de fin des épisodes événementiels sélectionnés de ce type d'événement environnemental.

7. Système de diffusion (1) par une montre (100) d'un message informatif relatif à une évaluation de la qualité du sommeil d'un porteur de ladite montre (100) mettant en oeuvre le procédé selon l'une quelconque des revendications précédentes, le système (1) comprenant les éléments suivants reliés entre eux : une unité de traitement (2), au moins un capteur environnemental (5) et une interface de diffusion d'une information visuelle et/ou sonore (3, 4).

8. Montre (100) comportant un système (1) selon la revendication précédente.

9. Montre (100) selon la revendication précédente, **caractérisée en ce qu'**elle est une montre (100) mécanique connectée.

10. Programme d'ordinateur comprenant des instructions de code de programme lesquelles, lorsque'elles sont exécutées par l'unité de traitement (2) dans un système de diffusion (1) selon la revendication 7 causent ledit système de diffusion (1) à exécuter le procédé selon l'une des revendications 1 à 6.

## Patentansprüche

1. Verfahren zum Übertragen einer Informationsnachricht, die sich auf eine Bewertung der Schlafqualität eines Trägers einer Uhr (100) bezieht, durch die Uhr (100), wobei das Verfahren die folgenden Schritte umfasst:
- Bestimmen des Beginns der Schlafphase des Trägers:
▪ anhand einer Beleuchtungsmessung von einem Umgebungssensor (5) der Uhr (100), wobei der Beginn der Schlafphase identifiziert wird, sobald eine schwache Beleuchtungsbedingung dann von der Verarbeitungseinheit (2) der Uhr bestimmt wird, und zwar dann, wenn die vom Sensor (5) übertragene Beleuchtungsmessung während eines bestimmten Zeitintervalls unter einem Lichtstärke-Referenzschwellenwert liegt; oder
▪ anhand von physiologischen Messdaten des Trägers und/oder von verhaltensbezogenen Messdaten des letzteren; oder
▪ anhand der Verwendung von zusätzlichen Informationen, wie etwa der Tageszeit und einer Schätzung des Schlaf-Wach-Rhythmus des Trägers, durch die Verarbeitungseinheit (2);
- als Reaktion auf das Bestimmen des Beginns der Schlafphase des Trägers Aufzeichnen (10) von beschreibenden Daten mindestens einer Ereignisepisode mindestens eines Typs von Umgebungsereignis, das während der Schlafphase des Trägers ausgelöst wurde, durch die Verarbeitungseinheit (2);
- Identifizieren (11) mindestens eines Typs von Umgebungsereignis, das den Schlaf des Trägers stört, durch ein Verarbeiten der beschreibenden Daten;
- Schätzen (16) eines Bewertungsindex der Schlafqualität des Trägers entsprechend eines Schlafstörungsindikators jedes Typs von identifiziertem schlafstörendem Umgebungsereignis, wobei in diesem Schritt (16) die Verarbeitungseinheit (2) den Bewertungsindex der Qualität dieses Schlafes bestimmt, indem sie einen Durchschnittswert des bzw. der Schlafstörungsindikatoren berechnet, die in einem Unterschritt des Erzeugens (14) eines Schlafstörungsindikators für jeden Typ von identifiziertem schlafstörendem Umgebungsereignis erhalten werden, wobei während dieser Berechnung Koeffizienten auf bestimmte Indikatoren entsprechend derArt des Typs von schlafstörendem Umgebungsereignis, auf das sie sich beziehen, angewendet werden können; und
- Entwerfen (17) der Informationsnachricht, die den geschätzten Bewertungsindex umfasst, zur Übertragung an den Träger.

2. Verfahren nach dem vorhergehenden Anspruch, **dadurch gekennzeichnet, dass** der Schritt des Identifizierens (11) einen Unterschritt des Auswählens (12) einer oder mehrerer Ereignisepisoden umfasst, die sich auf jeden Typ von Umgebungsereignis beziehen, das während der Schlafphase des Trägers anhand mindestens eines Auswahlkriteriums aufgezeichnet wurde.

3. Verfahren nach dem vorhergehenden Anspruch, **dadurch gekennzeichnet, dass** ein erstes Auswahlkriterium vorsieht, dass mindestens eine Umgebungsmessung der beschreibenden Daten jeder Ereignisepisode, die sich auf jeden Typ von Umgebungsereignis bezieht, mit einem Referenzschwellenwert der Schlafstörung verglichen wird.

4. Verfahren nach einem der Ansprüche 2 und 3, **dadurch gekennzeichnet, dass** ein zweites Auswahlkriterium darin besteht, eine Dauer jeder Ereignisepisode, die sich auf jeden Typ von Umgebungsereignis bezieht, mit einem Referenzschwellenwert der Dauer der Schlafstörung zu vergleichen.

5. Verfahren nach einem der vorhergehenden Ansprüche, **dadurch gekennzeichnet, dass** der Schritt des Identifizierens (11) einen Unterschritt des Erzeugens (14) eines Schlafstörungsindikators für jeden Typ von identifiziertem schlafstörendem Umgebungsereignis umfasst.

6. Verfahren nach dem vorhergehenden Anspruch, **dadurch gekennzeichnet, dass** der Unterschritt des Erzeugens (14) eine Phase (15) des Berechnens des Schlafstörungsindikators für jeden Typ von identifiziertem schlafstörendem Umgebungsereignis anhand der folgenden beschreibenden Merkmale umfasst:
- einen Mittelwert der Umgebungsmessung(en), die in den beschreibenden Daten der ausgewählten Ereignisepisoden, die sich auf diesen Typ von Umgebungsereignis beziehen, enthalten ist/sind;
- eine Gesamtdauer des Ablaufs dieses Typs von Umgebungsereignis;
- die Anzahl ausgewählter Ereignisepisoden dieses Typs von Umgebungsereignis;
- die Start- und Endzeiten der ausgewählten Ereignisepisoden dieses Typs von Umgebungsereignis.

7. System zum Übertragen (1) einer Informationsnachricht, die sich auf eine Bewertung der Schlafqualität eines Trägers der Uhr (100) bezieht, durch die Uhr (100), das das Verfahren nach einem der vorhergehenden Ansprüche durchführt, wobei das System (1) die folgenden Elemente umfasst, die miteinander verbunden sind: eine Verarbeitungseinheit (2), mindestens einen Umgebungssensor (5) und eine Schnittstelle zum Übertragen einer visuellen und/oder akustischen Information (3, 4).

8. Uhr (100), die ein System (1) nach dem vorhergehenden Anspruch umfasst.

9. Uhr (100) nach dem vorhergehenden Anspruch, **dadurch gekennzeichnet, dass** sie eine mechanische Smartwatch (100) ist.

10. Computerprogramm, das Programmcodeanweisungen umfasst, die, wenn sie durch eine Verarbeitungseinheit (2) in einem Übertragungssystem (1) nach Anspruch 7 ausgeführt werden, das Übertragungssystem (1) dazu veranlassen, das Verfahren nach einem der Ansprüche 1 bis 6 auszuführen.

## Claims

1. Method of transmission by a watch (100) of an information message relating to an assessment of the sleep quality of a user of said watch (100), the method including the following steps:
- determining the start of the user's sleep period:
- from a lighting measurement provided by an environmental sensor (5) of the watch (100), the start of the sleep period being identified once a low lighting condition has been determined by the processing unit (2) of the watch, and when the lighting measurement transmitted by the sensor (5) is below a reference light intensity threshold during a set time interval; or
- from physiological measurement data for the user and/or behavioural measurement data therefor; or
- from the use, by the processing unit (2), of additional information such as the time of day and an estimation of the circadian rhythm of the user;
- in response to the determination of the start of the user's sleep period, recording (10), by the processing unit (2), data describing at least one event-driven episode of at least one type of environmental event recorded during said sleep period of the user;
- identifying (11) at least one type of environmental event that disturbs the user's sleep by processing said descriptive data;
- estimating (16) a sleep quality assessment index for the user as a function of a sleep disturbance indicator for each identified type of sleep disturbing environmental event, wherein, during this step (16), the processing unit (2) determines the assessment index for the quality of this sleep by computing an average of the one or more sleep disturbance indicators obtained in a sub-step (14) of generating a sleep disturbance indicator for each identified type of sleep disturbing environmental event, during which computation coefficients can be applied to certain indicators as a function of the nature of the type of sleep disturbing environmental event to which they relate; and
- devising (17) an information message comprising the estimated assessment index for the purpose of diffusion to the user.

2. Method according to the preceding claim, **characterised in that** the identification step (11) comprises a sub-step (12) of selecting one or more event-driven episodes relating to each type of environmental event recorded during the sleep period of the user using at least one selection criterion.

3. Method according to the preceding claim, **characterised in that** a first selection criterion provides that at least one environmental measurement of the descriptive data of each event-driven episode relating to each type of environmental event is compared to a reference threshold of sleep disturbance.

4. Method according to any of claims 2 and 3, **characterised in that** a second selection criterion consists in comparing a duration of each event-driven episode relating to each type of environmental event to a reference threshold of duration of sleep disturbance.

5. Method according to any of the preceding claims, **characterised in that** the identification step (11) comprises a sub-step (14) of generating a sleep disturbance indicator for each identified type of sleep disturbing environmental event.

6. Method according to the preceding claim, **characterised in that** the generation sub-step (14) comprises a phase (15) of calculating the sleep disturbance indicator for each identified type of sleep disturbing environmental event using the following descriptive features:
- a mean value of the environmental measurement(s) comprised in the descriptive data of the selected event-driven episodes relating to this type of environmental event;
- a total duration of this type of environmental event;
- the number of selected event-driven episodes of this type of environmental event;
- the start and end times of the selected event-driven episodes of this type of environmental event.

7. System of transmission (1) by a watch (100) of an information message relating to an assessment of the sleep quality of a user of said watch (100) that implements the method according to any of the preceding claims, the system (1) comprising the following elements which are connected to each other: a processing unit (2), at least one environmental sensor (5) and an interface for diffusing visual and/or audio information (3, 4).

8. Watch (100) including a system (1) according to the preceding claim.

9. Watch (100) according to the preceding claim, **characterised in that** it is a connected mechanical watch (100).

10. Computer program comprising program code instructions which, when executed by the processing unit (2) in a transmission system (1) according to claim 7, cause said transmission system (1) to execute the method according to one of claims 1 to 6.
